# FASCICULE DE BREVET EUROPEEN

(11) **EP 1 881 792 B1**
(45) Date de publication et mention de la délivrance du brevet: **08.07.2009**
(21) Numéro de dépôt: 06755501.1
(22) Date de dépôt: 18.05.2006
(51) Int. Cl.: A61B 17/68, A61F 2/28

(54) **IMPLANT DESTINE A ETRE UTILISE AVEC UNE PLAQUE D'OSTEOTOMIE**
IMPLANTAT ZUR VERWENDUNG MIT EINER OSTEOTOMIEPLATTE
IMPLANT FOR USE WITH AN OSTEOTOMY PLATE

(30) Priorité: 20.05.2005 FR 0505084
(43) Date de publication de la demande: 30.01.2008
(73) Titulaire: D.L.P., 44690 La Haye Fouassiere (FR)
(72) Inventeur: LARCHÉ , Grégoire, F-49300 Cholet (FR)
(74) Mandataire: Tournel, Jean Louis
(86) Numéro de dépôt international: PCT/FR2006/001127
(87) Numéro de publication internationale: WO 2006/123057

(56) Documents cités:
- FR-A- 2 741 525
- US-A- 5 766 251
- US-A1- 2003 105 526
- US-A1- 2003 125 739

## Description

L'invention se rapporte à un implant destiné à être utilisé avec une plaque d'ostéotomie dans le cadre d'une correction de l'alignement des membres inférieurs par addition.

Elle vise plus particulièrement mais non limitativement l'ostéotomie tibiale de valgisation.

Le membre inférieur se compose du fémur du tibia/péroné et du pied.

Chez la majorité des sujets, il existe un alignement du fémur et du tibia qui permet à l'articulation du genou de travailler normalement et donc de passer les efforts.

Mais chez certains sujets, on peut diagnostiquer un genou valgum ou un genou varum ce qui aboutit à une usure irrégulière du cartilage du plateau tibial. Ce problème est accentué par le fait que les forces ne sont pas régulièrement reparties sur l'ensemble du plateau tibial.

En effet, il est connu que 70% des efforts passent par la partie interne du plateau tibial et donc 30% par la partie externe.

Il existe également une répartition entre l'avant et l'arrière qui est de l'ordre de 60% sur l'avant et 40 % sur la partie postérieure.

Du fait de cette répartition particulière et de différents problèmes tels que le surpoids, une usure anormale peut apparaître rapidement lorsque le patient est atteint d'un genou varum.

Il est donc conseillé d'intervenir avant qu'il soit nécessaire de placer une prothèse totale de genou.

Cette intervention consiste donc à réaligner le tibia avec le fémur par addition interne. On procède donc de la manière suivante pour la valgisation du tibia.

On réalise un trait d'ostéotomie dans la zone métaphysaire interne.

Cette coupe doit être précise et préserver une partie d'os qui sert de charnière externe (ostéoclasie).

Par une cale introduite dans le trait de coupe, on corrige angulairement l'axe du tibia par rapport au fémur pour le réaligner.

Il faut alors fixer cette correction par un moyen approprié le temps que l'os se régénère et se consolide.

On utilise généralement une plaque d'ostéotomie fixée sur la face interne du tibia. Cette plaque doit encaisser tous les efforts mécaniques le temps que l'os se forme et comble l'espace créé.

Il faut entre six à neuf mois pour que l'os repousse.

En position corrigée, les deux faces en vis à vis formées lors de la coupe sont éloignées et délimitent un volume en forme de coin.

On connaît une cale US 2003/0105526 qui s'étend sur la quasi totalité de la coupe et qui est destinée à rester définitivement en place. C'est uniquement l'os cortical qui va se régénérer si la cale est en acier inoxydable c'est pourquoi on utilise un matériau à cellules ouverte pour que l'os repousse partout.

Cette cale n'est donc pas destinée à être retirée.

On connait aussi une cale FR 2741525 en forme de fer à cheval qui utilise un matériau à cellule ouverte ou est simplement grillagé pour favoriser la colonisation osseuse. L'échancrure de la cale permettant le passage d'une tige tibiale.

Comme bien souvent, cette intervention retarde la mise en place d'une prothèse de genou, et pour que cette cale ne gêne pas lors de l'implantation d'une prothèse de genou, la cale est en forme de fer à cheval de manière à pouvoir insérer plus tard une queue de prothèse dans l'échancrure de la cale qui n'est pas extractible. Indépendamment de cette cale, pour combler plus rapidement cet espace en coin créé après résection, on introduit dans celui-ci de l'os naturel prélevé, par exemple, sur la crête iliaque ou un substitut osseux qui va agir notamment comme catalyseur en vue d'accélérer la formation de l'os.

Ce précurseur osseux est en contact par ses faces supérieure et inférieure avec les faces de l'os qui doivent se rejoindre.

On préfère généralement utiliser un substitut osseux car cela évite un geste chirurgical qui est nécessaire lorsqu'on prélève de l'os naturel. Ce substitut osseux va être de forme adaptée pour quasiment combler l'espace créé lors de l'ostéotomie. Ce substitut osseux couvre donc la corticale et l'os spongieux.

Il existe deux types de substituts osseux : l'un est très friable alors que l'autre est plus résistant.

Certains chirurgiens travaillent exclusivement avec du substitut dense et d'autres avec du substitut friable.

Le substitut osseux dense, donc plus résistant, se résorbe beaucoup moins rapidement que le substitut friable mais le substitut osseux dense n'est cependant pas conçu pour encaisser les efforts importants de compression.

Même lorsqu'on utilise du substitut dense, la remise en charge du tibia doit être très progressive car la fabrication de l'os reste relativement longue du fait de la colonisation des pores de ce substitut dense qui est moins rapide.

On constate malheureusement que certains patients ne respectent pas le protocole post-opératoire ce qui provoque des problèmes au niveau de la formation de l'os.

En effet, la plaque d'ostéotomie est conçue pour résister et transmettre les efforts mais comme tout matériau métallique, elle présente une certaine souplesse, dès lors les efforts sur la plaque d'ostéosynthèse génèrent des micros-mouvements induisant des forces de cisaillement entre les faces de l'os et celles du substitut.

Il n'y a donc pas une ostéo-conduction normale.

On constate alors une encapsulation avec une fibrose pour finir par une pseudo-arthrose.

Il faut alors recommencer l'opération.

Le chirurgien est alors gêné par le substitut osseux notamment lorsque celui-ci est dense.

Au lieu d'utiliser une plaque d'ostéotomie, il est donc connu d'utiliser un implant en forme de coin US 2003/0105526 dont l'étendue correspond sensiblement au volume cunéiforme qui est introduit dans l'espace ouvert lors de la valgisation. Ce coin est maintenu par des vis qui, traversant cet implant, sont vissées dans l'os.

Cet implant peut être en acier inoxydable, en titane, en céramique etc.

L'os va venir se former autour de cet implant qui occupe une grande partie du volume cunéiforme.

C'est cet implant qui va encaisser tous les efforts. Il n'est pas nécessaire de mettre une plaque d'ostéotomie

Du fait de l'absence de plaque d'ostéotomie, la remise en charge du tibia est très longue et l'os qui pousse va subir constamment des micro-traumatismes. On a donc les mêmes difficultés que dans le cas de la plaque d'ostéotomie.

Comme on l'a dit précédemment l'échancrure est destiné à pouvoir laisser passer une queue de prothèse. Elle n'est pas destinée à y loger un précurseur osseux.

On connaît également US 5766251 un coin en hydroxyapatite percé en son centre d'un canal permettant d'y insérer un précurseur osseux.

L'os va commencer à pousser au travers du canal puis ce coin est colonisé par l'os progressivement et ne peut être retiré. Par ailleurs au travers de ce canal l'os repousse et même si l'on souhaitait extraire ce coin, cela serait impossible.

La présence de ce coin présente une gêne lors de la pose d'une prothèse car la matière constituant cette cale n'a pas les mêmes propriétés que l'os.

On connaît également US2003/0125739 un implant pour vertèbre mais qui en place ne peut être retiré.

L'invention se propose d'apporter une solution aux différents problèmes évoqués ci-avant.

A cet effet, l'invention a pour objet un implant bio-inerte destiné à être utilisé avec une plaque d'ostéotomie dans le cadre d'une correction de l'alignement des membres inférieurs par addition, cet implant présentant un logement débouchant dans ses faces supérieure et inférieure, cet implant étant **caractérisé en ce que** qu'il a une résistance mécanique suffisante pour transmettre et/ou absorber les efforts auxquels il est soumis pour préserver le précurseur osseux placé dans le logement et il est extractible sans affaiblissement notable de la reconstruction osseuse et à cet effet le logement logeant le précurseur osseux a une forme d'échancrure et l'implant occupe un volume de l'ordre de 10 à 35% du foyer d'ostéotomie

L'invention sera bien comprise à l'aide de la description ci-après faite à titre d'exemple non limitatif en regard du dessin qui représente schématiquement :

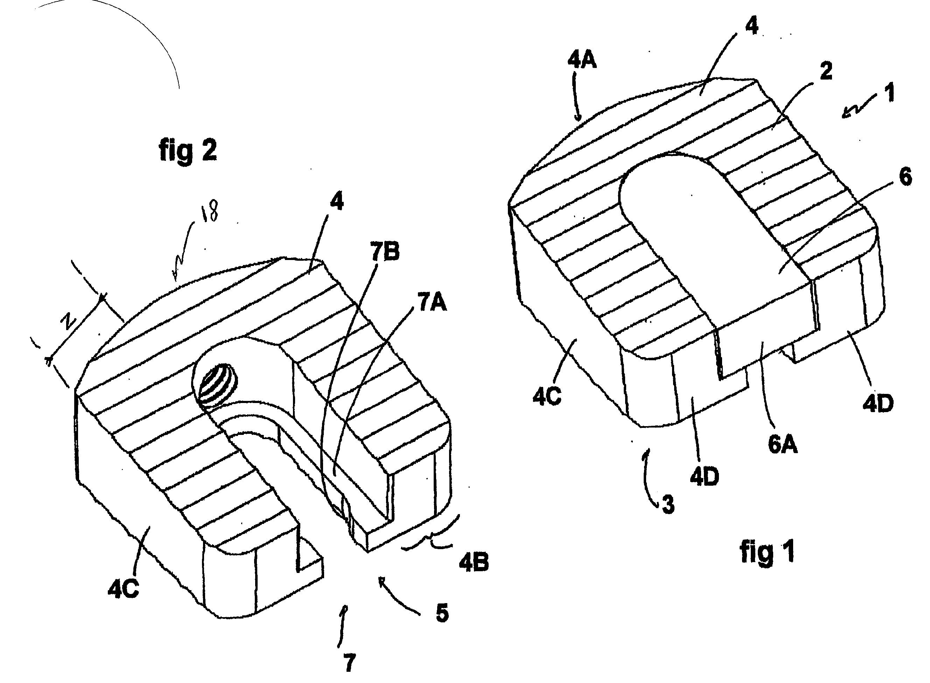

FIG 1 : un implant
FIG 2 : l'implant sans le substitut osseux interne

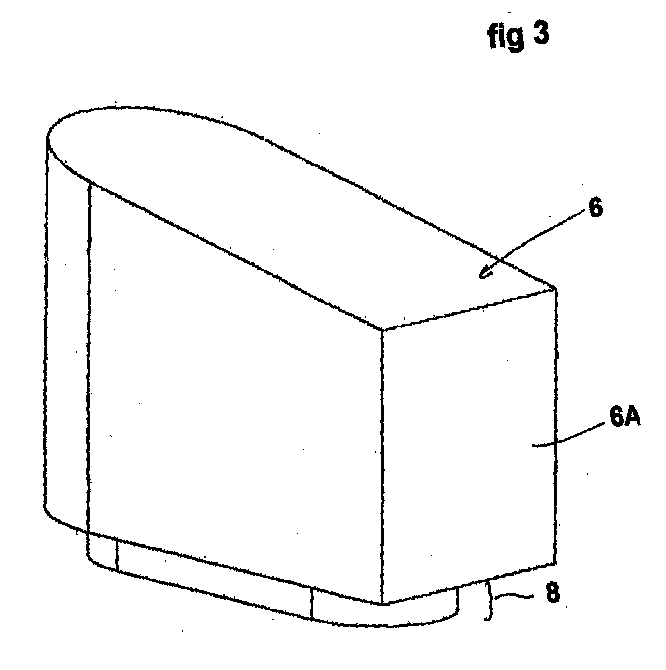

FIG 3 : Le substitut osseux interne qui vient se fixer sur la partie de l'implant de la figure 2.
FIG 4 : Tibia après ostéotomie et avant mise en place de l'implant et de la plaque d'ostéosynthèse.
FIG 5 : une variante d'implant
FIG 6 : Une représentation en transparence de l'implant et de la plaque d'ostéotomie posés

En se reportant au dessin, on voit que pour corriger l'axe des membres inférieurs, on pratique une ostéotomie de valgisation par addition.

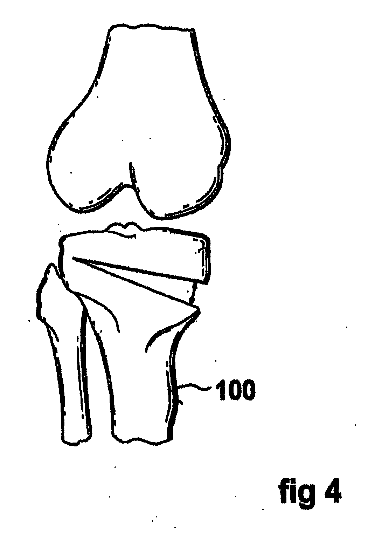

On a représenté en figure 4 une ostéotomie tibiale de valgisation.

Après avoir procédé à l'ostéotomie, on réoriente le tibia 100 au moyen d'une cale (non représentée) adaptée à l'angle choisi pour la correction.

On voit très bien l'angle formé après correction.

On fixe l'orientation du membre avec une plaque d'ostéotomie 200 fixée sur la face antéro-inteme avec des vis puis, après avoir retiré la cale, on introduit l'implant 1. Cet implant est en matériau bio-inerte et peut comporter un activateur de la repousse de l'os.

Cet implant est provisoire, c'est à dire qu'il est retiré après que l'os se soit reformé.

Il est en matériau bio inerte de sorte à ne pas être colonisé.

Le volume de cet implant est largement inférieur au volume du foyer d'ostéotomie. Il est de l'ordre de 10 à 35% du volume du foyer d'ostéotomie, les faces supérieure et inférieure de l'implant étant en contact avec l'os.

De préférence, ce volume est de l'ordre de 10 à 20% du volume du foyer d'ostéotomie. La surface occupée est très faible. A titre d'exemple, la surface occupée est généralement de l'ordre de deux à trois centimètres carré.

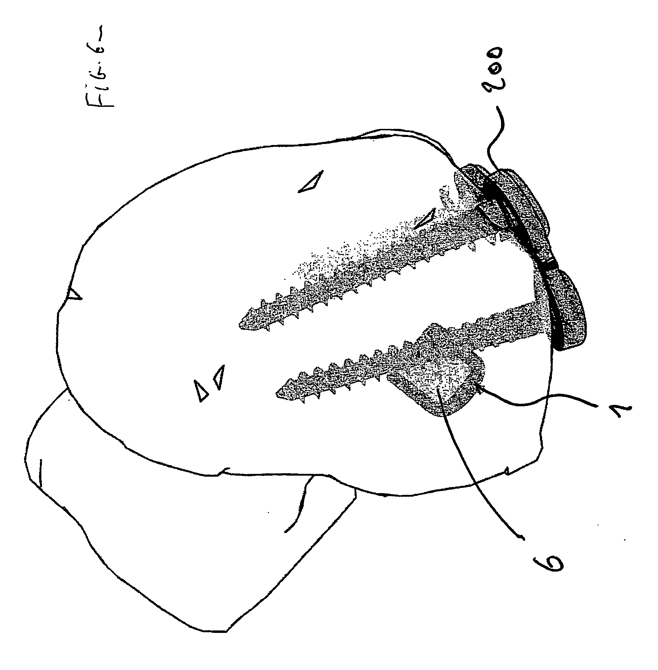

On voit très bien en figure 6 que la taille de cet implant est réduite.

La longueur de l'implant est inférieure au rayon de la section de l'os mesuré au niveau de la coupe. Il est généralement posé au niveau du ligament latéral interne, le bord arrière étant à la rase de la corticale . Dans un plan frontal, l'implant ne dépasse jamais le plan passant par le massif interne des épines tibiales.

L'implant 1 à une épaisseur décroissante depuis sa paroi externe pour s'insérer dans l'espace cunéiforme créé par l'ostéotomie et le changement angulaire du tibia.

Les faces supérieure 2 et inférieure 3 de l'implant 1 déterminent des plans sécants dont l'angle correspond sensiblement à la correction souhaitée afin que les faces en vis à vis du foyer d'ostéotomie s'appliquent sur les faces supérieure et inférieure de l'implant.

L'implant présente un logement A débouchant dans ses faces supérieure et inférieure et une résistance mécanique suffisante pour transmettre et/ou absorber les efforts auxquels il est soumis.

La résistance mécanique de cet implant va s'ajouter à la résistance mécanique de la plaque d'ostéotomie et donc limiter les micro-déplacements.

Le temps de sa mise en place, l'implant protége le précurseur osseux.

De préférence, ces caractéristiques mécaniques seront proches de celles de la corticale de l'os.

Par exemple, il s'agira d'un polymère éventuellement armé de fibres de carbone qui aura pour avantage d'être radio-transparent.

Par exemple, il pourra s'agir de polyetheretherketone connu sous le sigle « PEEK » Avantageusement, le logement A reçoit un activateur de repousse de l'os ou précurseur osseux tel de l'os prélevé ou un substitut osseux. Cet activateur de repousse occupe environ 70% du volume total de l'implant.

De par sa résistance mécanique, l'implant va ainsi constituer une protection destinée à protéger l'activateur de repousse tel le substitut osseux des efforts induits par la mise en charge du membre.

En effet, la paroi de l'implant peut subir les contraintes ce qui stabilise la zone où se positionne le précurseur osseux.

Les efforts vont donc être repris en majorité par la plaque d'ostéotomie fixée latéralement mais cet implant participera à une stabilisation.

L'implant comprend une partie 4 externe ayant une forme en fer à cheval délimitant ainsi une échancrure 5 dans laquelle se loge un précurseur 6 osseux friable. L'ouverture de cette échancrure 5 débouche dans la zone de plus faible épaisseur de l'implant.

Cette partie 4 en fer à cheval est délimitée par une face 4A dorsale courbe et deux parois 4B latérales dont les faces externes 4C sont planes.

Notamment pas sa faible occupation du site d'ostéotomie, cet implant peut être retiré facilement sans affaiblir notamment l'os repoussé.

L'os repoussé dans l'échancrure n'empêche pas le retrait de l'implant. En effet le logement étant ouvert latéralement, l'implant peut glisser et laisser passer cet os formé au niveau du logement.

Les faces 4D avant de ces parois latérales sont planes et définissent un plan contenant ultérieurement la face 6A dite avant du substitut 6 osseux lorsqu'il sera mis en place. Cet implant 1 sera positionné en sorte que l'arrière de la partie en fer à cheval se situe au niveau de la corticale et l'entrée de l'échancrure vers le centre.

Les faces latérales internes de l'échancrures sont, de préférence, parallèles ainsi que les faces latérales externes afin de faciliter l'extraction de l'implant après repousse de l'os.

La dimension Z transversale de la paroi du fer à cheval est comprise entre 2 et 6 millimètres. Elle est de l'ordre de l'épaisseur de la corticale.

Cette partie 4 en forme de fer à cheval sera en un matériau bio-inerte présentant des caractéristiques mécaniques beaucoup plus élevées que celle du substitut 6 osseux friable.

Cet implant comprend des moyens 7 en vue de maintenir un substitut osseux dans le logement.

Pour maintenir ce substitut, l'échancrure du fer à cheval présente, par exemple, au niveau de sa base, un rebord 7A sur lequel s'appuie le substitut osseux interne.

La base 8 du substitut 6 osseux interne est section réduite en sorte de pouvoir s'emboîter dans la section délimitée par la face interne du rebord 7A, par exemple, sur une hauteur égale à l'épaisseur du rebord précité.

Le précurseur osseux est légèrement serré dans l'échancrure.

Dans une variante, au moins l'une des faces supérieure et inférieure de la partie 4 de l'implant en forme de fer à cheval est striée en sorte de limiter les glissements dans l'os.

Les stries seront limitées à la zone où se situe l'os spongieux c'est à dire au niveau des branches de l'échancrure.

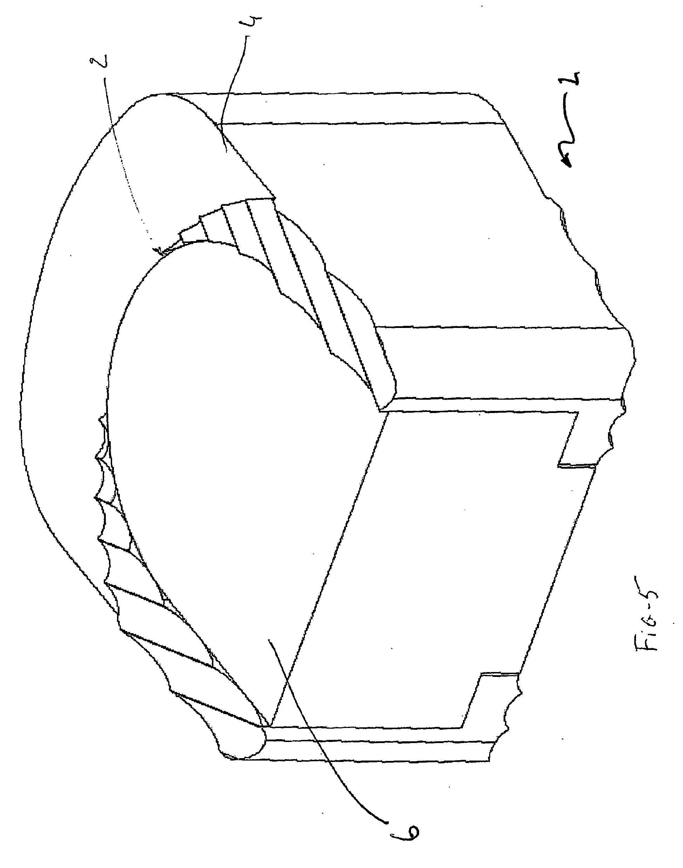

La partie arrière de l'implant ne sera pas striée, cette partie arrière est celle qui se positionne au niveau de la corticale. Ces stries ont une orientation inclinée par rapport à un plan vertical médian de symétrie. Ces stries ont une orientation en miroir comme on peut le voir sur la figure 5.

L'avantage de cette solution est que la partie 4 externe de l'implant apporte une résistance supplémentaire à la plaque d'ostéosynthèse et évite que le substitut osseux soit sollicité.

La partie 4 externe a un effet protecteur car elle va absorber les contraintes.

Cet implant va donc transférer les contraintes du bas vers le haut et inversement comme doit le faire la plaque d'ostéosynthèse en préservant des contraintes le substitut osseux qui pourra jouer son rôle sans perturbation.

Au niveau du dos de la partie en fer à cheval, il est prévu un moyen 18 pour ancrer un ancillaire en vue de retirer facilement cet implant.

Par exemple, on a prévu un trou taraudé.

La forme en fer à cheval facilitera l'extraction de cet implant par translation selon une direction sensiblement perpendiculaire à la face d'entrée du fer à cheval.

Ainsi comme on le comprend, cet implant est provisoire et vise à protéger le précurseur osseux mis en place le temps que l'os repousse. La présence d'une plaque d'ostéotomie est nécessaire car cet implant n'a pas vocation a reprendre toute le poids du corps. La forme échancrée permet de le retirer facilement et sa petite taille évite, lorsque cet implant est retiré, d'affaiblir la zone de reconstruction osseuse.

## Revendications

1. Implant en matériau bio-inerte *de sorte à ne pas être colonisé par la repousse de l'os* avec précurseur osseux destiné à être utilisé avec une plaque d'ostéotomie dans le cadre d'une correction de l'alignement des membres inférieurs par addition, cet implant présentant un logement débouchant dans ses faces supérieure et inférieure ledit implant ayant une résistance mécanique suffisante pour transmettre et/ou absorber les efforts auxquels il est soumis pour préserver le précurseur osseux placé dans ledit logement et cet implant étant, d'une part, extractible sans affaiblissement notable de la reconstruction osseuse, le précurseur osseux étant logé dans le logement qui a une forme d'échancrure *avec une ouverture latérale permettant à l'implant d'être extrait par translation après repousse de l'os dans le logement*, et *l'implant qui a une forme de fer à cheval* occupe un volume de l'ordre de 10 à 35% du foyer d'ostéotomie et d'autre part, il comprend des moyens (7) en vue de maintenir le précurseur osseux dans ledit logement.

2. Implant selon la revendication 1 **caractérisé en ce que** l'échancrure présente au niveau de sa base, un rebord (7A) sur lequel s'appuie le précurseur osseux.

3. Implant selon la revendication 2 **caractérisé en ce que** la base (8) du précurseur osseux est de section réduite en sorte de pouvoir s'emboîter dans la section délimitée par la face interne du rebord (7A).

4. Implant selon la revendication 1 **caractérisé en ce que** le précurseur osseux est légèrement serré dans l'échancrure.

5. Implant selon la revendication 1 **caractérisé en ce que** les caractéristiques mécaniques de l'implant sont proches de celles de la corticale de l'os.

6. Implant selon la revendication 1 ou 2 **caractérisé en ce que** le matériau constituant l'implant est un polymère.

7. Implant selon la revendication 1 **caractérisé en ce que** l'échancrure est délimitée par une face (4A) dorsale courbe et deux parois (4B) latérales dont les faces externes (4C) sont planes formant ainsi un fer à cheval.

8. Implant selon la revendication 1 **caractérisé en ce que** la dimension (Z) transversale de la paroi en fer à cheval de l'implant est comprise entre 2 et 6 millimètres.

9. Implant selon la revendication 1 **caractérisé en ce qu'**au niveau du dos de la partie en fer à cheval est prévu un moyen (18) pour ancrer un ancillaire en vue d'extraire l'implant

10. Implant selon la revendication 6 **caractérisé en ce que** le polymère est du polyethyletherketone connu sous le sigle « PEEK » .

## Claims

1. Implant made of bio-inert material *in order to avoid being colonized by bone re-growth* with the bone precursor destined to be used with an osteotomy plate to correct alignment of the lower limbs by addition, this implant having a housing which opens into its upper and lower faces, said implant having a mechanical strength sufficient for transmitting and/or absorbing the strain to which it is subjected in order to protect the bone precursor which is placed in said housing and this implant being, on the one hand, removable without significant weakening of bone reconstruction, the bone precursor being placed in the housing which has a notch shape *with a lateral opening enabling the implant to be removed by displacement after re-growth of bone in the housing, and the implant, which is horseshoe-shaped,* occupies a volume of around 10 to 35% of the osteotomy area and, on the other hand, it includes means (7) of keeping the bone precursor in said housing.

2. Implant according to claim 1 **characterised in that** the notch presents at its base, a raised edge (7A) on which the bone precursor is supported.

3. Implant according to claim 2 **characterised in that** the base (8) of the bone precursor is a smaller section so that it is able to be fitted into the section defined by the inner side of the raised edge (7A).

4. Implant according to claim 1 **characterised in that** the bone precursor is slightly tightened in the notch.

5. Implant according to claim 1 **characterised in that** the mechanical features of the implant are close to those of the bone cortex.

6. Implant according to claim 1 or 2 **characterised in that** the material making up the implant is a polymer.

7. Implant according to claim 1 **characterised in that** the notch is defined by a curved dorsal face (4A) and two lateral walls (4B) of which the external faces (4C) are flat, thus forming a horseshoe shape.

8. Implant according to claim 1 **characterised in that** the transverse aspect (Z) of the horseshoe-shaped wall of the implant covers between 2 and 6 millimetres.

9. Implant according to claim 1 **characterised in that** at the back of the horseshoe section provision has been made for a means (18) to anchor an ancillary tool with a view to removing the implant.

10. Implant according to claim 6 **characterised in that** the polymer is polyethyletherketone known under the acronym "PEEK".

## Patentansprüche

1. Implantat aus blo-inertem Werkstoff, solcher Art, der durch Nachwachsen des Knochens nicht besiedelt wird, mit einem Knochenvorläufer, das im Rahmen einer Korrektur der Ausrichtung unterer Extremitäten in öffnender additiver Technik zur Verwendung mit einer Osteotomie-Platte bestimmt ist, wobei das Implantat eine an seiner Ober- und Unterseite offene Aufnahme aufweist, wobei das Implantat eine ausreichende mechanische Festigkeit hat, um die Belastungen, denen es ausgesetzt ist, zu übertragen und/oder auszunehmen, um den in die Aufnahme eingebrachten Knochenvorläufer zu schützen, und wobei das Implantat einerseits ohne erhebliche Schwächung des Knochenwiederaufbaus entnehmbar ist, wobei der Knochenvorläufer in die Aufnahme eingefügt ist, welche die Form eines bogenförmigen Ausschnitts mit einer seitlichen Öffnung hat, durch die das Implantat nach dem Nachwachsen des Knochens in der Aufnahme translatorisch entnommen werden kann, und das Implantat, das die Form eines Hufeisens hat, ein Volumen von etwa 10 bis 35 % des Osteotomieraums einnimmt, und es andererseits Mittel (7) zum Halten des Knochenvorläufers in der Aufnahme umfässt.

2. Implantat nach Anspruch 1, **dadurch gekennzeichnet, dass** der bogenförmige Ausschnitt in Höhe seiner Basis einen Rand (7A) aufweist, auf dem der Knochenvorläufer aufliegt.

3. Implantat nach Anspruch 2, **dadurch gekennzeichnet, dass** die Basis (8) des Knochenvonäufers einen kleineren Querschnitt hat, um in den von der Innenseite des Rands (7A) umgrenzten Querschnitt eingesteckt werden zu können.

4. Implantat nach Anspruch 1, **dadurch gekennzeichnet, dass** der Knochenvorläufer in den bogenförmigen Ausschnitt leicht eingeklemmt ist.

5. Implantat nach Anspruch 1, **dadurch gekennzeichnet, dass** die mechanischen Eigenschaften des Implantats denen der Kortikalls des Knochens ähnlich sind.

6. Implantat nach Anspruch 1 oder 2, **dadurch gekennzeichnet, dass** der das Implantat bildende Werkstoff ein polymer ist.

7. Implantat nach Anspruch 1, **dadurch gekennzeichnet, dass** der bogenförmige Ausschnitt von einer gebogenen Rückseite (4A) und zwei Seitenwänden (4B) umgrenzt ist, deren Außenseiten (4C) eben sind, die somit ein Hufeisen bilden.

8. Implantat nach Anspruch 1, **dadurch gekennzeichnet, dass** das Querschnittsmaß (Z) der hufeinsenförmigen Seitenwand des Implantats 2 bis 6 Millimetern beträgt.

9. Implantat nach Anspruch 1, **dadurch gekennzeichnet, dass** in Höhe der Rückseite des hufeisenförmigen Teils ein Mittel (18) zum Verankern eines chirurgischen Instruments vorgesehen ist, um das Implantat zu entnehmen.

10. Implantat nach Anspruch 6, **dadurch gekennzeichnet, dass** das Polymer aus Polyetheretherketon ist, bekannt unter der Abkürzung PEEK.
